# EUROPÄISCHE PATENTSCHRIFT

(11) **EP 1 665 106 B1**
(45) Veröffentlichungstag und Bekanntmachung des Hinweises auf die Patenterteilung: **09.11.2011**
(21) Anmeldenummer: 04765128.6
(22) Anmeldetag: 13.09.2004
(51) Int. Cl.: G06F 19/00

(54) **VERFAHREN UND PROGRAMM ZUR ZAHNÄRZTLICHEN ARBEITSVORBEREITUNG**
METHOD AND PROGRAM FOR THE PREPARATION OF DENTAL WORK
PROCEDE ET PROGRAMME DE PREPARATION D'UN TRAVAIL DENTAIRE

(30) Priorität: 11.09.2003 EP 03020686
(43) Veröffentlichungstag der Anmeldung: 07.06.2006
(73) Patentinhaber: Pfeiffer, Manfred, Dubai (AE)
(72) Erfinder: Pfeiffer, Manfred, Dubai (AE)
(74) Vertreter: Szynka, Dirk
(86) Internationale Anmeldenummer: PCT/EP2004/010206
(87) Internationale Veröffentlichungsnummer: WO 2005/027010

## Beschreibung

Zahnärzte führen bei der Behandlung des Gebisses von Patienten komplexe technische Verfahren aus, die neben der eigentlichen zahnärztlichen Untersuchung, Behandlung und Kontrolle teilweise auch andere technische Berufsgruppen, etwa Zahntechniker und andere Spezialisten für die Herstellung individuell angepasster technischer Produkte und Laborarbeiten, beinhalten. Diese Verfahren und die zugehörige Arbeitsvorbereitung werden immer anspruchsvoller. Auch werden Datenverarbeitungsgeräte und Computerprogramme zunehmend in Zahnarztpraxen eingesetzt, um die zu bestimmten Behandlungskomplexen gehörenden Daten zu erfassen und zu verwalten.

Im Folgenden wird die zahnärztliche Tätigkeit im Hinblick auf ihre Eigenschaft als technische Tätigkeit betrachtet. Zwar ist die Behandlung des menschlichen Körpers selbst von der Patentierung mangels gewerblicher Anwendbarkeit ausgeschlossen, jedoch ist sie ohne Zweifel technisch.

Gleiches gilt für die zugehörige Arbeitsvorbereitung, also die Ablaufplanung einschl. Projektskizzierung, Konstruktion, Prototypenherstellung etc., die wie bei der Herstellung komplexer technischer Erzeugnisse ebenfalls einen Teil technischer Herstellungsvertahren bildet.

Der Stand der Technik Turner, P. J. et al. "Basic Computing for Dental Practitioners: 5. Practice Management Systems", Dental Update, 1998, Bd. 25, Seiten 332 - 338, behandelt Zahnarztpraxis-Management-Systeme, mit denen Sekretariatsarbeiten, administrative und Buchhaltungs-Aufgaben schneller und genauer ausgeführt werden sollen, wobei insbesondere eine Computeraltemative zu Terminkalendem angesprochen wird, die die Verfügbarkeit des Arztes und des Patienten berücksichtigt und Übersichtlichkeit schaffen soll.

Zudem beschreibt auch Mack K. "Moving Forward To Total Integration", Hawaii Dental Journal, 2002, Bd. 33, Nr. 3, Seiten 13 - 15, die Fortschritte der Einführung einer effizienten Datenverarbeitung in Zahnarztpraxen, insbesondere im Hinblick auf die computergestützte Verwaltung von Behandlungsterminen.

Der vorliegenden Erfindung liegt das technische Problem zugrunde, verbesserte Möglichkeiten zur technischen Arbeitsvorbereitung bei der zahnärztlichen Tätigkeit zur Verfügung zu stellen.

Die Erfindung richtet sich auf ein Verfahren zur zahnärztlichen Arbeitsvorbereitung mit dem Schritt:
Aufnahme von Daten zu einem zahnärztlichen Behandlungsprojekt in ein Datenverarbeitungsgerät,
gekennzeichnet durch die zusätzlichen Schritte:
   Abrufen von vorbestimmten Ablauftabellendaten aus einem Ablauftabellenspeicher des Datenverarbeitungsgeräts, welche Ablauftabellendaten den Behandlungsprojektdaten zugeordnet sind und technische Details im Sinne vorbestimmter zeitliche Parameter zu zu dem Behandlungsprojekt gehörenden Behandlungsschritten enthalten, welche vorbestimmten zeitlichen Parameter die zeitliche Reihenfolge von Behandlungsschritten in dem Behandlungsprojekt wiedergeben,
   Erstellen eines Behandlungsablaufplans aus den Ablauftabellendaten, welcher eine Mehrzahl Behandlungseinheiten mit jeweiligen Terminen enthält, wobei den Behandlungseinheiten jeweilige Behandlungsschritte zugeordnet sind und die Zuordnung der Behandlungsschritte zu den Behandlungseinheiten und den Terminen die in den Ablauftabellendaten enthaltenen vorbestimmten zeitlichen Parameter berücksichtigt, sowie auf ein entsprechendes Computerprogrammprodukt und auf ein entsprechend programmiertes Datenverarbeitungsgerät.

In der folgenden Beschreibung wird zwischen diesen verschiedenen Patentkategorien nicht mehr im Einzelnen unterschieden, so dass die Offenbarung im Hinblick auf das Verfahren, das Computerprogrammprodukt und auch das Datenverarbeitungsgerät zu verstehen ist.

Die Erfindung geht davon aus, dass bei der zahnärztlichen Arbeitsvorbereitung zunächst Daten zu einem zukünftigen Behandlungsprojekt anfallen, etwa weil der Zahnarzt bei der Untersuchung eines Patienten festgestellt hat, dass ein bestimmter Zahn mit einer Brücke und benachbarte Zähne mit einer Krone zu versehen sind. Der Begriff Behandlungsprojekt meint dabei in einem gemeinsamen Planungszusammenhang geplante und abgewickelte Behandlungsschritte.

Das erfindungsgemäße Verfahren sieht vor, dass die zu dem Behandlungsprojekt gehörenden Daten, die also beispielsweise eine Brücke und zwei Kronen an benachbarten Zähnen kennzeichnen, zum Aufsuchen und Abrufen von zugeordneten Ablauftabellendaten aus einem Ablauftabellenspeicher in einem Datenverarbeitungsgerät genutzt werden. In diesen Ablauftabellendaten sind die technischen Einzelheiten der zu dem Behandlungsprojekt gehörenden Behandlungsschritte abgelegt, wobei zur Veranschaulichung auf das Ausführungsbeispiel verwiesen wird. Insbesondere enthalten die Ablauftabelleridaten als wesentlichen Unterschied zu konventionellen Zahnarzt-Verwaltungsprogrammen zeitliche Parameter zu den Behandlungsschritten.

Konventionelle Programme haben sich nämlich darauf beschränkt, die für eine einheitliche und korrekte Rechnungserstellung erforderlichen Informationen zusammenzustellen und ggf. selbsttätig einen Heil- und Kostenplan oder eine Rechnung zu erzeugen. Im Unterschied dazu soll die Erfindung auch in die technische Arbeitsvorbereitung des Zahnarztes eingebunden sein. Dazu enthalten die Ablauftabellendaten technische Details der Behandlungsschritte im Sinne zeitlicher Parameter, und zwar zumindest Parameter, die die zeitliche Reihenfolge von Behandlungsschritten innerhalb des Behandlungsprojekts wiedergeben.

Damit ist es in dem erfindungsgemäßen Verfahren möglich, dem Zahnarzt bei der Arbeitsvorbereitung bereits programmseitig eine vorbereitete Übersicht über die anfallenden Behandlungsschritte in der richtigen zeitliche Reihenfolge und u. U. auch, soweit zutreffend, in der richtigen zeitlichen Zusammengehörigkeit vorzugeben. Der Zahnarzt kann also in ein vorgefertigtes Schema von zeitlich geordneten Behandlungsschritten eingreifen und jeweilige Behandlungssitzungen mit jeweiligen Terminen festlegen. Er ist bei seiner Arbeitsvorbereitung nicht nur dadurch unterstützt, dass die Gefahr gemindert wird, wichtige oder zumindest optional sinnvolle Behandlungsschritte zu vergessen (bei entsprechend anspruchsvoll gepflegten Ablauftabellendaten kann hier auch eine echte Vollständigkeit sichergestellt werden) - es sind zudem technische, d. h. hier die medizinische und die technische Arbeitsvorbereitung betreffende zeitliche Zusammenhänge im Sinne von zeitlichen Reihenfolgen und Gleichzeitigkeiten berücksichtigt und damit entsprechende Fehler ausgeschlossen.

Mit diesen Ablauftabellendaten kann das erfindungsgemäße Verfahren bzw. Programm dann einen Behandlungsablaufplan erstellen, der eine Mehrzahl Behandlungseinheiten mit jeweiligen Terminen enthält. Diese Behandlungseinheiten können Behandlungssitzungen sein, zu denen der Patient die Praxis aufsucht. Es kann sich auch um bestimmte zeitlich festgelegte Behandlungsschritte oder Gruppen von in zeitlichem Zusammenhang auszuführende Behandlungsschritten handeln, die ohne den Patienten in der Praxis, im zuarbeitenden Labor etc. auszuführen sind. Dieser Behandlungsablaufplan berücksichtigt dabei in der Zuordnung der Behandlungsschritte zu den Behandlungseinheiten die genannten zeitlichen Parameter. Die eigentlichen Termine können dabei je nach Ausführungsform vom Zahnarzt oder Praxispersonal, ggf. nach Rücksprache mit den Patienten oder dem Labor, eingetragen oder auch teilweise vom Programm selbsttätig erzeugt werden, etwa wenn bestimmte nicht mit dem Patienten direkt abzustimmende zeitliche Schemata vorab feststehen.

Verkürzt und vereinfacht ausgedrückt sieht die Erfindung also vor, zeitliche Zusammenhänge und die zeitliche Dimension der Arbeitsvorbereitung als technisch relevante Größe in ein Zahnarztprogramm einzuführen und dieses Programm damit für die Arbeitsvorbereitung nutzbar zu machen. Dies ist von besonderer Bedeutung, weil der technische Fortschritt in der Zahnheilkunde zunehmend komplexere Abläufe mit sich bringt und die zeitlichen Zusammenhänge nicht ohne weiteres leicht und fehlerfrei zu überschauen sind. Darüber hinaus muss die Zusammenarbeit verschiedener behandelnder Spezialisten, neben dem Zahnarzt beispielsweise auch der Kieferchirurg, eine spezielle Assistenz oder der Dentaltechniker, und die Beschaffung und Bereitstellung von Material und Hilfsmitteln koordiniert werden.

Vor allem geht die Tendenz zu "Generalsanierungen" des Gebisses, also umfassenden und mehr oder weniger vollständigen Behandlungsprojekten, die im Erfolgsfall lediglich eine Erhaltung des dadurch gewonnen Zustands nach sich ziehen. Andererseits wechselwirken verschiedene Behandlungsschritte am Patienten und insbesondere auch Behandlungsschritte unter Einbeziehung der außerhalb der eigentlichen Arbeit am Patienten selbst stattfindenden Teile der Behandlung stark miteinander, bedingen sich gegenseitig, behindern sich gegenseitig oder können in effizienter Weise gemeinsam durchgeführt werden.

Der Begriff "Behandlungsschritt" meint hier übrigens jeden einzelnen mit der zahnärztlichen Behandlung in Zusammenhang stehenden Teil des Behandlungsprojekts, also auch Zuarbeiten des Labors, Arbeiten des Kieferchirurgen und Anderes, was nicht zur Arbeit des Zahnarztes am Patienten selbst gehört. Der Begriff "Behandlungsschritte" umfasst insbesondere auch reine Untersuchungen und Kontrollen, bei denen also der Patientenkörper oder für diesen vorgesehene Produkte nicht im eigentlichen Sinn manipuliert werden.

Insbesondere sind zwischen verschiedenen Behandlungsschritten häufig relevante Mindestzeitabstände zu beachten, etwa technisch bedingte Mindestvorbereitungs- und Arbeitszeiten eines Labors oder Technikers, Mindesteinheilzeiten bis zur Belastbarkeit, Mindestausheilzeiten entzündlicher Prozesse nach einer Behandlung und dgl. Bei einer bevorzugten Ausführungsform sieht die Erfindung vor, dass die zeitlichen Parameter in den Ablauftabellendaten zumindest wesentliche der Mindestzeitabstände zwischen Behandlungsschritten beinhalten und das Programm konsequenterweise reagiert, wenn diese Mindestzeitabstände bei der zeitlichen Planung des Behandlungsprojekts, also bei der Terminzuordnung zu den Behandlungseinheiten, nicht beachtet werden.

Das Programm kann bei dieser Reaktion eine Warnanzeige in Form eines Ausdrucks, einer Bildschirmanzeige oder eines Tonsignals geben oder auch eine (durch den Benutzer aufhebbare) Sperrung der Variation durchführen.

Es ist bevorzugt, dass der Behandlungsablaufplan durch einen Benutzer, etwa den Zahnarzt oder sein Praxispersonal, interaktiv variiert werden kann. Ein Beispiel ist die manuelle Termineingabe. Allerdings umfasst die Erfindung auch Ausführungsformen, bei denen die Terminvergabe ggf. in Abstimmung mit anderen Organisationsprogrammen selbsttätig erfolgt und jedenfalls Änderungen, die die genannten zeitlichen Parameter nicht einhalten würden, nicht vorgenommen werden können.

Insbesondere ist bevorzugt, dass der Benutzer den Behandlungsablaufplan im Laufe der Ausführung des Behandlungsprojekts abhängig von Untersuchungs- und Behandlungsergebnissen variiert und ihn das Programm vorzugsweise auch zur Bestätigung bzw. Änderung einzelner Teile des Behandlungsprojekts abhängig von solchen Ergebnissen auffordert.

Ferner ist bevorzugt, dass für einzelne Behandlungseinheiten, insbesondere Behandlungssitzungen mit dem Patienten, Checklisten erstellt und ausgegeben werden, insbesondere auf einem Papierbogen ausgedruckt oder einem Bildschirm angezeigt werden. Dem Zahnarzt oder Praxispersonal, der/das u. U. eine unmittelbar computergestützte Arbeit nicht gewohnt ist, kann damit in der vertrauten Weise anhand von listenartigen Vorgaben einzelne Behandlungseinheiten durchführen, ohne sich mit dem Behandlungsprojekt insgesamt in dem Datenverarbeitungsgerät befassen zu müssen.

In ähnlicher Weise ist bevorzugt, Terminmerkblätter insbesondere für den Patienten zu erstellen und auszugeben, um eine Einhaltung der vergebenen Termine in konventioneller Form zu gewährleisten.

Ferner können das erfindungsgemäße Verfahren und Programm die Behandlungsprojektdaten neben den bereits geschilderten Aspekten der Arbeitsvorbereitung auch zahnärztlichen Gebührenordnungsziffern oder Ziffern von Laborpreislisten, wie etwa der GOÄ, der GOZ, der BEB/BEL, zuordnen und damit eine Einbeziehung der buchhalterischen Aspekte des Behandlungsprojekts oder eine Verzahnung mit einer diesbezüglichen Verwaltung erleichtern. Insbesondere kann das Programm selbsttätig Rechnungsvorschläge einschl. Laborrechnungsvorschlägen und/oder Kostenpläne erstellen.

Es wurde bereits ausgeführt, dass der Behandlungsablaufplan durch einen Benutzer interaktiv variiert werden kann. Eine wichtige Ausführungsform der Erfindung sieht vor, dass der Behandlungsablaufplan oder zumindest ein Teil desselben mit entsprechenden einzelnen Behandlungsschritten durch das Datenverarbeitungsgerät ausgegeben wird. Diese Ausgabe kann insbesondere in Form einer Monitoranzeige oder eines Ausdrucks erfolgen, etwa auf einer Karteikarte oder einem einfachen Blatt Papier.

Daraufhin werden bei dieser Ausführungsform Daten zu dem ausgegebenen Teil des Behandlungsablaufplans handschriftlich eingegeben. Handschriftlich bedeutet hier eine durch die Hand geführte Bewegung eines Schreibgeräts, aber nicht notwendigerweise ein Schreiben im klassischen Sinn mit Farbstoff auf Papier. Handschriftlich beinhaltet beispielsweise auch das handgeführte Schreiben auf einem berührungsempfindlichen Monitor. Diese handschriftliche Eingabe soll auf dem ausgedruckten oder im Monitor angezeigten Behandlungsablaufplan erfolgen, also in den Ausdruck bzw. die Anzeige "hinein". Daraufhin erkennt das Datenverarbeitungsgerät die eingegebenen Daten entweder durch elektronische Auswertung im Fall des Schreibens auf dem berührungsempfindlichen Monitor oder durch Einscannen eines Ausdrucks. Es sind übrigens dabei auch Mischfälle denkbar, etwa ein Ausdruck auf einem Träger, der dann auf einer die Handschrift erfassenden Unterlage beschriftet wird, so dass das Einscannen entfallen kann.

Im einfachsten Fall werden die handschriftlichen Daten einfach im Sinne ihres Vorhandenseins in bestimmten Feldern erfasst. Dazu enthält die Ausgabe (was begrifflich Ausdruck und Monitoranzeige umfassen soll) Leerfelder, also beschreibbare Bereiche, wobei eine feste Beziehung zwischen dem Ort dieser Bereiche und der Bedeutung der Beschriftung vorgegeben ist. Beispielsweise kann eine Beschriftung in bestimmten leeren Feldern als Abhaken verstanden werden und eine Beschriftung in anderen von dem Datenverarbeitungsgerät bereits beschrifteten Feldern als Durchstreichen.

Bei einer bevorzugten und aufwendigeren Variante soll jedoch die Handschrift im Sinne einer Schrifterkennung (OCR, Optical Character Recognition) als eigentliche Schrift gelesen werden, was vor allem bei Eingabe in Druckbuchstaben keine wesentlichen technischen Schwierigkeiten mit sich bringt. Auch im Fall einer eigentlichen Schrifterkennung ist es übrigens bevorzugt, dass ein formloses Durchstreichen zulässig bleibt und als solches erkannt wird, dass also beispielsweise bei einer über einen bestimmten Schwellenwert hinausgehenden Beschriftung eines eigentlich bereits maschinenbeschriebenen Feldes dieses als Streichung des Inhalts des Feldes, etwa Streichung eines Behandlungsschritts, gewertet wird.

Das Programm bietet damit zum einen eine gute Information des behandelnden Zahnarztes durch Vorlage eines ein Übersehen oder Versehen vermeidenden Behandlungsablaufplans, also eine Gewährleistung einer Lege Artis-Behandlung. Beispielsweise kann das Programm den Zahnarzt sicherheitshalber veranlassen, vor einer Überkronung eines Zahnes eine Vitalitätsprobe vorzunehmen. Würde diese nämlich aus Nachlässigkeit unterbleiben, wäre dies ein Behandlungsfehler. Es kann darüber hinaus in besonders einfacher Weise und damit für den behandelnden Zahnarzt oder einem Mitarbeiter besonders effizient Rückkopplungen, also Korrekturen, Bestätigungen, Ergänzungen des Behandlungsablaufplans, ermöglichen. Diese können in der beschriebenen handschriftlichen Weise quasi wie mit dem Umgang mit einem Notizzettel erfolgen und damit auch von Personen, die mit dem Umgang mit Datenverarbeitungsgeräten keine Erfahrung haben oder dazu nicht neigen, leicht durchgeführt werden. Dabei ergeben sich für die handschriftliche Dateneingabe natürlich alle zuvor bereits erwähnten Möglichkeiten, etwa die Veränderung von Einzelheiten des Behandlungsablaufplans, beispielsweise eine Terminverlegung.

Wenn die Ausgabe als Ausdruck erfolgt, ist es besonders bevorzugt, diesen Ausdruck außerhalb des entsprechenden Behandlungszimmers zu erzeugen. Bei konventionellen Zahnarztverwaltungsprogrammen haben sich viele Nutzer gegen eine Bedienung in den Behandlungszimmern gewehrt. Die Erzeugung eines Ausdrucks und die Möglichkeit der handschriftlichen Rückkopplung gemäß der Erfindung bieten damit eine für den Zahnarzt und das Personal bequeme Möglichkeit der Einbeziehung des Programms und einer Unterstützung durch dasselbe, ohne deswegen im Behandlungszimmer selbst mit einem Datenverarbeitungsgerät umgehen zu müssen.

Die Erfindung lässt sich aber natürlich auch in dem Behandlungszimmer selbst ausführen, insbesondere bei einer Monitoranzeige als Ausgabe. Dann kann die Handbeschriftung auf einem entsprechenden "touch-screen-Monitor" infolge der günstigen Ergonomie solche Hemmungen bei den Nutzern abbauen helfen. Das Einscannen des beschrifteten Ausdrucks kann wiederum außerhalb des Behandlungszimmers erfolgen.

Bei der Verwendung von eigentlichen Ausdrucken ist es ferner bevorzugt, diese mit einem Barcode zu versehen, der eine Dokumentenidentifikation, insbesondere eine Identifikation des Behandlungsablaufplans oder des entsprechenden Teils dazu, ermöglicht. Dieser Barcode kann bei dem beschriebenen Einscannen eine schnelle Vorabzuordnung ermöglichen, bei Bedarf aber auch unabhängig davon durch Abfahren mit einem optischen Lesestift schnell klären, zu welchem Patienten bzw. zu welchem Behandlungsprojekt der entsprechende Ausdruck gehört.

Ferner ist im Falle eines Ausdrucks vorzugsweise vorgesehen, dass der Ausdruck einen Bereich enthält, der beim Einscannen gar nicht mehr berücksichtigt wird. Dies fördert die Geschwindigkeit und reduziert die zu verarbeitende Datenmenge. Dieser Bereich kann für handschriftliche Bemerkungen und Notizen genutzt werden, die nicht in das Programm eingegeben werden müssen. Die entsprechenden Ausdrucke mit diesen Notizen können dann eine weitergehende Behandlungsdokumentation ermöglichen, insbesondere wenn es sich dabei um Karteikarten klassischer Form handelt, mit denen in Zahnarztpraxen üblicherweise dokumentiert wird.

Der Ausdruck kann zur Erleichterung des Einscannens des tabellarischen Aufbaus Zuordnungslinien enthalten. Die Zuordnungslinien können insbesondere eine durch Linien gezeichnete Kästcheneinteilung bedeuten, aber auch von der Maschinenbeschriftung in dem Ausdruck eingehaltene Begrenzungslinien sein. Damit kann bei dem Einscannen eine leichtere örtliche Zuordnung, insbesondere eine Mittenbestimmung oder Parallelausrichtung erfolgen.

Bei der Schrifterkennung, ob nun durch Einscannen oder in anderer Weise, kann es zu Erkennungsunsicherheiten kommen. Bevorzugt ist hier, dass die Anzeige des erkannten Behandlungsablaufplans (oder -teils) auf dem Monitor solche Problembereiche optisch markiert, insbesondere durch farbige Hinterlegung des entsprechenden Feldes.

Die handschriftliche Eingabe kann eine Bestätigung oder Autorisierung durch den Zahnarzt selbst beinhalten. Damit wird quasi festgehalten, dass ein in dem Behandlungsablaufplan enthaltener Behandlungsschritt tatsächlich als Leistung erbracht worden ist. Nur in solcher Form bestätigte Behandlungsschritte werden tatsächlich zur Abrechnung gebracht. Bevorzugt ist hierbei, dass der Zahnarzt diese Bestätigung durch oder verbunden mit einer handschriftliche Datumseingabe zum Zeitpunkt der Leistungserbringung vornimmt. Insbesondere kann die Ausgabe, auf der diese handschriftliche Bestätigung erfolgt, das entsprechende Datum des Behandlungsablauf plans, das im Normalfall also mit dem einzutragenden Datum übereinstimmen sollte, verschweigen, also freilassen. In diesem Fall kann das Programm gegebenenfalls auch auf Abweichungen hinweisen, ist jedenfalls aber das Fehlen des Datums plausibler Anlass für die Ausfüllung, die dann wegen der Abwesenheit eines ausgegebenen Datums größeren Authentizitätswert hat. Ferner kann natürlich ein handschriftliches Namenskürzel oder Symbol durch den Zahnarzt eingetragen werden, das zum einen Signaturcharakter haben kann und zum zweiten in Gemeinschaftspraxen eine Zuordnung zu dem behandelnden Arzt erlauben kann.

Ein weiterer Aspekt der Erfindung liegt darin, dass das Programm, übrigens auch unabhängig von der Ausgabe von Behandlungsablaufplanteilen und deren Handbeschriftung, anhand der Ablauftabellendaten und möglicherweise weiterer eingegebener Daten zu dem Behandlungsprojekt Schätzungen für den Zeit- und Materialaufwand vornehmen kann. Aufgrund dieser Schätzungen kann ermittelt werden, mit welchem Abrechnungsfaktor sich ein ausreichender Rechnungsbetrag unter Zugrundelegung der entsprechend anzuwendenden Gebührenordnungsziffem ergibt. Sollte dieser Abrechnungsfaktor dann eine beispielsweise durch eine Krankenkasse vorgegebene Grenze überschreiten, kann das Datenverarbeitungsgerät automatisch einen entsprechenden Ausdruck ausgeben und damit den Zahnarzt zu einer schriftlichen Patientenvereinbarung über die entsprechende erhöhte Berechnung veranlassen.

Die Erfindung stellt sich zum einen als technisches Arbeitsverfahren dar. Des weiteren bezieht sich die Erfindung natürlich auf ein dieses Verfahren ermöglichendes Computerprogramm in in ein Datenverarbeitungsgerät geladener Form, auf einen Datenträger gespeicherter Form oder irgendeiner anderen Form. Schlussendlich bezieht sich die Erfindung auch auf ein mit dem erfindungsgemäßen Computerprogramm versehenes Datenverarbeitungsgerät, das dementsprechend an das erfindungsgemäße Verfahren angepasst ist. Der Begriff "Datenverarbeitungsgerät" umfasst dabei nicht nur Einzelgeräte sondern auch komplexe Datenverarbeitungsanlagen, Rechnernetze und dgl.

Die Erfindung wird im Übrigen anhand des folgenden Ausführungsbeispiels näher erläutert, wobei die dabei offenbarten Einzelmerkmale auch in anderen Kombinationen erfindungswesentlich sind und die Beschreibung natürlich nur beispielhaft erfolgt, also nicht den Gegenstand der Erfindung einschränkt.

Im Einzelnen zeigt:
- Fig. 1: eine schematische Übersicht über den Verfahrensablauf bei der zahnärztlichen Behandlung eines Patienten nach konventioneller Vorgehensweise;
- Fig. 2: einen beispielhaften Verfahrensablauf eines Behandlungsprojekts zur Verdeutlichung von Zeitparametem der Behandlungsschritte;
- Fig. 3: einen zweiten beispielhaften Verfahrensablauf eines Behandlungsprojekts;
- Fig. 4: einen dritten beispielhaften Verfahrensablauf eines Behandlungsprojekts;
- Fig. 5: ein schematisches Diagramm zur Verdeutlichung der Arbeitsweise des erfindungsgemäßen Programms;
- Fig. 6: einen beispielhaften Bildschirminhalt des in Fig. 5 dargestellten Programms;
- Fig. 7: eine analog Fig. 1 aufgebaute Darstellung des Verfahrensablaufs gemäß der Erfindung;
- Fig. 8: eine beispielhafte Ausgabe eines sitzungsbezogenen Behandlungsablauf planteils;
- Fig. 9: dieselbe Ausgabe einschließlich handschriftlicher Eingaben;
- Fig. 10: die Ausgabe aus Figur 8 in eingescannter Form im rechten Teil mit der nach Erkennung folgenden Monitoranzeige im linken Teil.

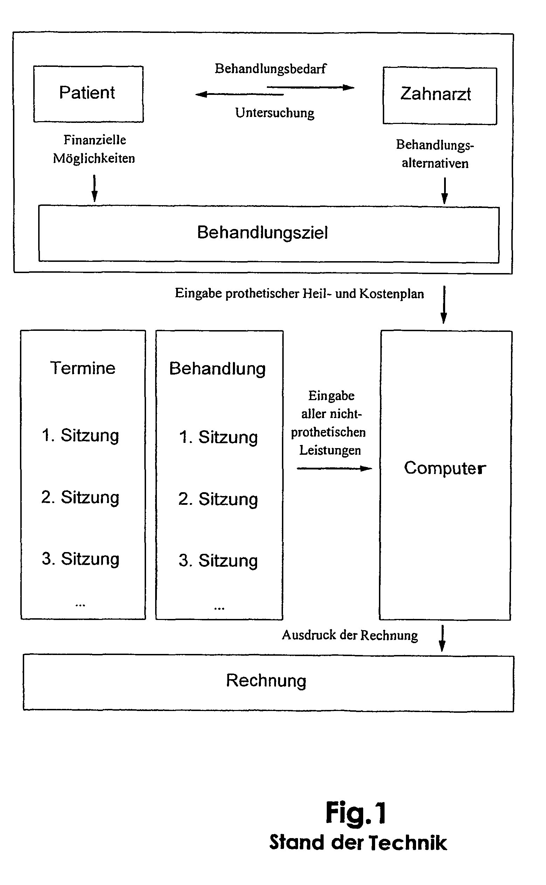

Üblicherweise erfolgt die Benutzung eines Zahnarzt-Computerprogramms in Zusammenhang mit der zahnärztlichen Behandlung bislang wie in Fig. 1 schematisch dargestellt. Der Patient vermittelt dem Zahnarzt seine Beschwerden und seine Vor geschichte und soweit für ihn erkennbar seinen Behandlungsbedarf. Der Zahnarzt führt eine erste Untersuchung durch, auf deren Basis in Abstimmung mit den finanziellen Möglichkeiten des Patienten zwischen verschiedenen Behandlungsalternativen ausgewählt wird und ein Behandlungsziel definiert wird. Dieses Behandlungsziel ist die dem Patienten vermittelte Zusammenfassung eines Behandlungsprojekts, das im Folgenden ablaufen soll.

Das Behandlungsprojekt gliedert sich in vielen Fällen in eine Vorbehandlung, eine prothetische Versorgung sowie eine Nachkontrolle und evtl. Korrekturen. Für den prothetischen Teil wird ein Heil- und Kostenplan erstellt, der den Hauptanteil der Gesamtkosten abdeckt. Dies erfolgt über ein Computerprogramm, dem auch während dem Ablauf des Behandlungsprojekts über verschiedene Behandlungseinheiten weitere Leistungen mitgeteilt werden. Die Behandlungseinheiten sind hier eine erste, eine zweite und eine dritte Sitzung mit verschiedenen Terminen.

Die Erstellung des Zeitplans für die Terminierung der Behandlung erfolgt unabhängig von dem Computerprogramm. Das Computerprogramm dient vielmehr zur Sammlung der Behandlungsdaten zwecks Erstellung von Heil- und Kostenplänen und zwecks Erleichterung der Rechnungserstellung, evtl. vollautomatischer Rechnungserstellung.

Wesentlich an diesem Stand der Technik ist, dass die zeitliche Planung der einzelnen Sitzungen unabhängig vom Heil- und Kostenplan erfolgt und das Computerprogramm die erbrachten Leistungen nur aus buchhalterischen Gründen sammelt und nur buchhalterisch zwischen diesen Leistungen differenzieren kann. Das Computerprogramm unterstützt also nicht bei der Arbeitsvorbereitung sondern lediglich bei der Buchhaltung und Abrechnung.

Andererseits bestehen zunehmend komplexe Zusammenhänge zwischen einzelnen Behandlungsschritten in Behandlungsprojekten des Zahnarztes. Zum Ersten ist dies durch den technischen Fortschritt in der Zahnheilkunde bedingt, zum Zweiten sind zunehmend verschiedene Personen innerhalb der Zahnarztpraxis und auch außerhalb der Zahnarztpraxis in ihrer Zusammenarbeit zu koordinieren und zum Dritten besteht ein Trend zu den bereits erwähnten "Generalsanierungen".

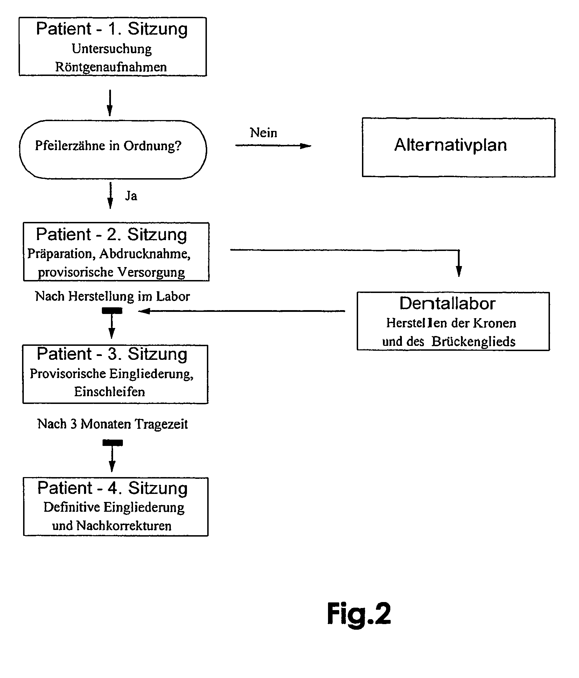

Der Übersichtlichkeit halber sollen hier keine besonders komplexen Beispiele dargestellt werden. Stattdessen zeigt Fig. 2 ein vereinfachtes Schema für ein einfacheres Behandlungsprojekt, nämlich die Eingliederung einer Brücke im Unterkiefer.

In einer ersten Behandlungssitzung wird der Patient untersucht und werden evtl. bereits Röntgenaufnahmen vorgenommen. Nach diesen kann entschieden werden, ob die als Pfeiler für die Brücke dienenden Zähne dafür geeignet sind und im negativen Fall ein Alternativplan aufgestellt werden, der hier nicht näher ausgeführt wird. Im positiven Fall wird mit dem Patienten eine zweite Sitzung vereinbart, bei der eine Präparation, eine Abdruckentnahme sowie eine provisorische Versorgung erfolgt.

Der Abdruck wird einem Dentallabor zur Verfügung gestellt, das dementsprechend Kronen und das Brückenglied herstellt. Diese Herstellung im Labor erfordert eine gewisse technische Bearbeitungszeit. Beispielsweise könnte hier eine Bearbeitungszeit von acht Arbeitstagen in Betracht kommen. Mit einem dementsprechenden zeitlichen Mindestabstand ist dann eine dritte Behandlungssitzung mit dem Patienten vereinbart worden, in der eine provisorische Eingliederung und ein Einschleifen erfolgt. Der Patient muss nun eine gewisse Tragezeit hinter sich bringen. Beispielsweise kann eine Zeit von drei Monaten angebracht sein, um einer evtl. traumatisierten Pulpa die notwendige Ausheilung zu ermöglichen. Die dreimonatige Tragezeit gibt also einen weiteren zeitlichen Mindestabstand zwischen der dritten Behandlungssitzung und einer darauf folgenden vierten Behandlungssitzung vor, in der eine definitive Eingliederung und evtl. Nachkorrekturen erfolgen.

Im vorliegenden einfachen Beispiel kommen als Zeitparameter also die technische Bearbeitungszeit des Dentallabors als Mindestabstand zwischen zweiter Behandlungssitzung und dritter Behandlungssitzung sowie die dreimonatige Ausheilzeit als Mindestzeitabstand zwischen dritter Behandlungssitzung und vierter Behandlungssitzung und zudem die technisch bedingte Reihenfolge dieser Behandlungsteile in Betracht.

Andererseits sollten provisorische Versorgungen in Abhängigkeit von der Fertigungsqualität nicht übermäßig lange im Mund des Patienten verbleiben, weil sonst deren weiterer Halt nicht gewährleistet ist und die Gefahr einer Kariesbildung entstehen kann. Daher ist ein maximaler Zeitabstand zwischen der zweiten Behandlungssitzung und der dritten Behandlungssitzung von vier Wochen als Beispielswert sinnvoll. Auch wenn dieser Maximalzeitabstand in vielen Fällen nicht zum Tragen kommen wird, weil ohnehin nach Patientenwunsch in möglichst rascher Folge Termine vergeben werden, so kommt es doch gerade bei berufstätigen Patienten immer wieder zu Absagen oder auch zu erst nachträglich erkannten Terminüberschneidungen in der Praxis. Werden dann neue Termine vereinbart, so ist es von erheblichem technischen Vorteil, zu gewährleisten, dass der Zeitabstand zwischen der zweiten Behandlungssitzung und der dritten Behandlungssitzung nicht übermäßig groß wird.

Analoge Argumente gelten für den Zeitabstand zwischen der dritten Behandlungssitzung und der vierten Behandlungssitzung. Hier sollte beispielsweise eine Tragezeit des nur provisorisch eingegliederten Kronen-/Brückensystems von einem Monat nicht überschritten werden.

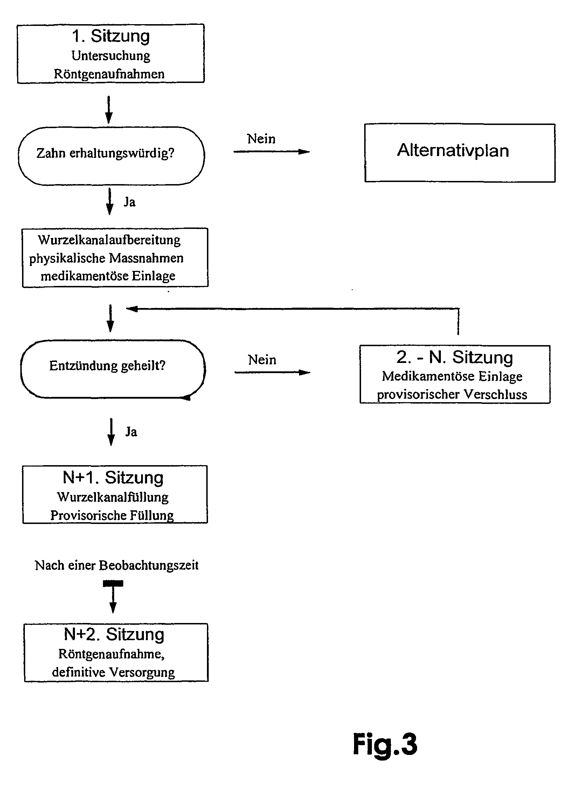

Ein weiteres Beispiel zur Veranschaulichung zeitlicher Parameter zeigt Fig. 3. Hier geht es um die konservierende Wurzelbehandlung bei einer grangänösen Pulpa. Die zerstörte Pulpa muss entfernt werden und der Wurzelkanal danach aufbereitet und gefüllt werden.

In einer ersten Behandlungssitzung erfolgt wieder eine Untersuchung und nach Möglichkeit bereits die Bewertung von Röntgenaufnahmen. Wie im vorherigen Beispiel soll auf einen im negativen Fall aufzustellenden Alternativplan nicht eingegangen werden. Im positiven Fall kommt es zur Wurzelkanalaufbereitung, zu hier nicht näher auszuführenden physikalischen Maßnahmen und einer medikamentösen Einlage, mit der die Entzündung beseitigt werden soll.

Nach einem gewissen zeitlichen Mindestabstand von drei Tagen wird nun untersucht, ob die akute Entzündung abgeheilt ist. Dieser zeitliche Mindestabstand ist technisch sinnvoll, weil eine vorzeitige Untersuchung mit nur sehr geringer Wahrscheinlichkeit zu einem positiven Ergebnis führen wird. Andererseits ist auch ein maximaler zeitlicher Abstand zu berücksichtigen, weil der aufbereitete Wurzelkanal noch nicht gefüllt ist. Hier kommen etwa sechs Monate in Betracht. Bei der ersten Behandlungssitzung zur Überprüfung wird nun entschieden, ob die Entzündung ausgeheilt ist. Im negativen Fall kommt es zu einer zweiten oder auch weiteren Sitzung zur Wiederüberprüfung, möglicherweise mit weiterer medikamentöser Einlage und provisorischem Verschluss. Nach dieser ersten Überprüfungssitzung und nach weiteren Sitzungen können wiederum zeitliche Mindestabstände und Maximalabstände beachtet werden von beispielsweise drei Tage bzw. sechs Monaten.

Wenn die Entzündung ausgeheilt ist, kann in derselben Behandlungssitzung oder in einer neu zu vereinbarenden Behandlungssitzung dann die Wurzelkanalfüllung erfolgen. Nach einer Beobachtungszeit von mindestens sechs bis neun Monaten erfolgt eine weitere Untersuchung, insbesondere eine Röntgenaufnahme und im positiven Fall eine Versorgung der Zahnkrone.

Dieses Beispiel verdeutlicht bereits eine größere Zahl von zeitlichen Parametern im Sinne von minimalen und maximalen Zeitabständen zwischen einzelnen Behandlungsschritten bzw. einzelnen Behandlungseinheiten. Es versteht sich, dass keine verbindlichen Aussagen darüber getroffen werden können, in welchen Fällen Mindestzeitabstände und in welchen Fällen Maximalzeitabstände zu beachten sind und wann nicht. Dies ist zum einen eine Frage der Sorgfalt des behandelnden Zahnarztes und zum anderen auch eine Frage der interindividuellen Variabilität der Patienten und der im Einzelnen verwendeten Materialien.

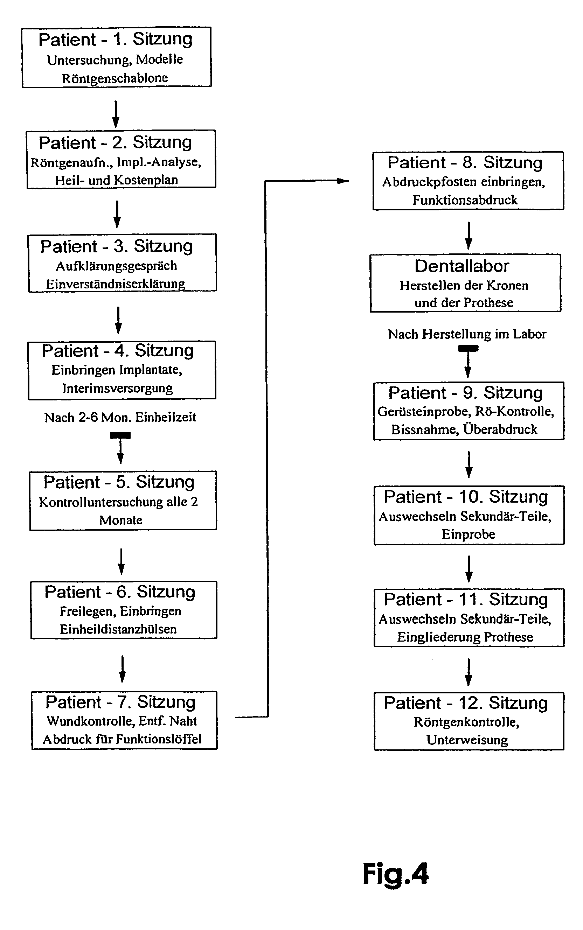

Ein drittes Beispiel in Fig. 4 zeigt schematisch den Behandlungsablauf bei einer sog. Cover-Denture-Prothese im Unterkiefer, die auf Implantaten verankert wird.

In einer ersten Behandlungssitzung erfolgt eine Untersuchung und die Erstellung von Modellen zur Fertigung einer Röntgenschablone. In einer zweiten Behandlungssitzung erfolgen Röntgenaufnahmen, eine Implantatanalyse sowie die Erstellung eines Heil- und Kostenplans. In einer dritten Behandlungssitzung kommt es dann zu einem detaillierten Aufklärungsgespräch mit Einverständniserklärung des Patienten, so dass in einer vierten Behandlungssitzung die Implantate eingebracht werden können. Nach einer im Bereich von zwei bis vier Monaten für Unterkiefer und sechs bis neun Monaten für Oberkiefer liegenden Mindesteinheilzeit erfolgt eine Kontrolluntersuchung im Rahmen einer fünften Behandlungssitzung, woraufhin in einer sechsten Behandlungssitzung die Implantate chirurgisch freigelegt und Einheildistanzhülsen eingebracht werden.

In einer siebten Behandlungssitzung erfolgt, wiederum mit einem bestimmten zeitlichen Mindestabstand zur sechsten Behandlungssitzung von einer Woche eine Wundkontrolle, die Entfernung der Naht und ein Abdruck für Funktionslöffel, woraufhin nach einem weiteren zeitlichen Mindestabstand von zwei Wochen in einer achten Behandlungssitzung Abdruckpfosten eingebracht werden können und ein Funktionsabdruck erfolgen kann. Daraufhin stellt das Dentallabor in einer typischen Bearbeitungszeit von drei Wochen die Kronen und Prothesen her, so dass es mit einem entsprechenden zeitlichen Mindestabstand zu einer neunten Behandlungssitzung mit dem Patienten kommt, die eine Gerüsteinprobe, eine Röntgenkontrolle, eine Bissnahme und einen Überabdruck beinhaltet.

In einer zehnten Behandlungssitzung werden Sekundärteile ausgewechselt, und es erfolgt eine Einprobe, in einer elften Behandlungssitzung werden wiederum Sekundärteile ausgewechselt, und es wird die Prothese eingegliedert, woraufhin nach einem weiteren Zeitabstand in einer zwölften Behandlungssitzung eine Röntgenkontrolle mit Patientenunterweisung erfolgt.

Die oben dargestellte Behandlung erstreckt sich über mindestens acht Monate, wobei nicht nur die Arbeiten des Zahnarztes, sondern auch die eines Kieferchirurgen und des Dentallabors sowie die Material- und Hilfsmittelbeschaffung zeitlich und fachlich zu koordinieren sind. Sie veranschaulicht den durch eine computergestützte Arbeitsvorbereitung und -koordinierung erzielbaren Qualitäts- und Sicherheitsgewinn. Andererseits sollte der Behandlungszeitraum nicht über achtzehn Monate im Oberkiefer und neun Monate im Unterkiefer hinausgehen, weil die Trabekelstruktur des Knochens sich erst unter Belastung bildet. Diese Maximalzeit betrifft genaugenommen den Abstand zwischen vierter und elfter Sitzung.

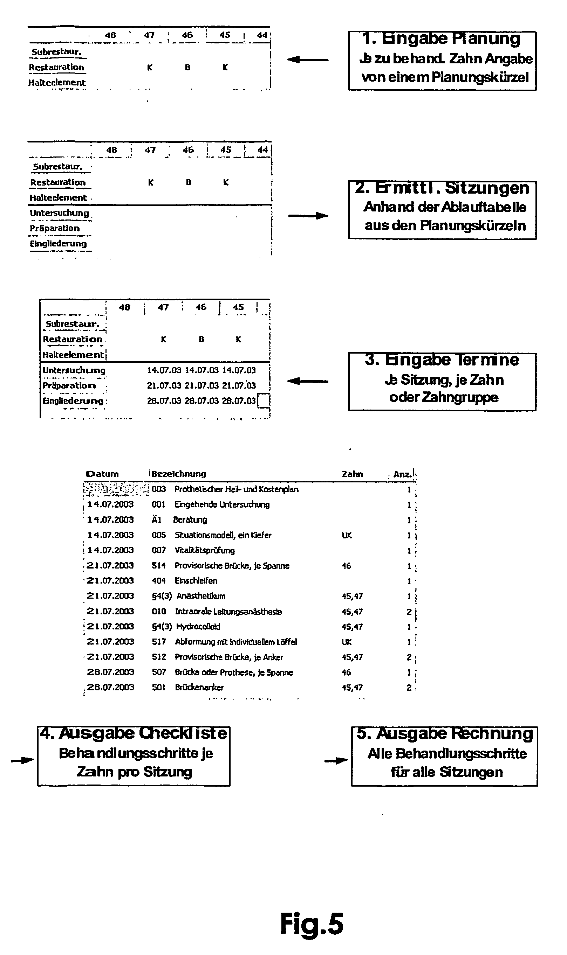

Fig. 5 veranschaulicht nun, wie hierbei das erfindungsgemäße Computerprogramm genutzt werden kann. Zunächst werden in einem in Fig. 5 oben links ausschnittsweise dargestellten Matrixschema sog. Planungskürzel für jeden zu behandelnden Zahn eingegeben. Dies entspricht der bisher so bezeichneten Aufnahme von Daten zu dem Behandlungsprojekt. Beispielsweise werden hier für den (aus Sicht des Patienten) unteren rechten Kieferteil die Zähne 5 - 7, also nach üblicher Nummerierung die Nummern 45 - 47, im Sinne einer Restauration eine Krone, ein Brückenglied und eine weitere Krone hierfür als Planungskürzel K, B, K eingegeben. Die Planungskürzel als aufgenommene Daten dienen dem Programm dazu, in vorbestimmten Ablauftabellen eines Ablauftabellenspeichers entsprechende Ablauftabellendaten zu den Planungskürzeln abzurufen. Beispielsweise werden für die Kronen und das Brückenglied jeweils Felder für Behandlungssitzungen zur Untersuchung, zur Präparation und zur Eingliederung geöffnet, wie das Diagramm zeigt. Das Programm kennt anhand der Ablauftabellendaten aber überdies die einzelnen Behandlungsschritte zu diesen Behandlungsteilen. '

In einem nächsten Schritt kann der Zahnarzt einer ersten Behandlungssitzung einen Termin zuweisen, hier den 14.07.2003 für eine Untersuchung in Bezug auf alle drei Zähne 45 - 47. Im weiteren Verlauf kann entweder das Programm zu den folgenden Terminen für die Präparation und die Eingliederung, also zu den folgenden beiden Behandlungsteilen, Terminfenster vorgeben, die vorgegebene Mindest- und Maximalzeitabstände berücksichtigen, oder frühest mögliche Termine angeben, die vom Zahnarzt nach Absprache mit dem Patienten verschoben werden können, oder auch nur eine frei vom Zahnarzt eingegebene Terminwahl auf die Einhaltung zeitlicher Parameter prüfen. Beispielsweise kann dies hier so erfolgen, dass nach Eingabe des 14.07.2003 für den zur Untersuchung gehörenden Behandlungsteil das Computerprogramm den zweiten Termin für die Präparation zur freien Eingabe vorgibt. Hier hat der Zahnarzt nun den 21.07.2003 eingegeben. Daraufhin kann das Programm unter Beachtung einer Mindestbearbeitungszeit von einer Woche für die Herstellung der Kronen und Brücke den 28.07.2003 als Eingliederungstermin vorschlagen, der in diesem Fall vom Zahnarzt und Patienten akzeptiert wurde. Bein einer Verschiebung hätte das Programm einen maximalen Zeitabstand zwischen Präparationstermin und Eingliederungstermin von beispielsweise vier Wochen berücksichtigt.

Das untere große Feld in Fig. 5 zeigt nun beispielhaft verschiedene Behandlungsschritte, die den verschiedenen Behandlungsteilen, also Sitzungen, zugeordnet sind. Diese Behandlungsschritte sind mit vor dem Klartext stehenden Kurzbezeichnungen bereits entsprechenden Kostenziffem oder Kostenvorschriften zugeordnet und werden in den beiden letzten Spalten bestimmten Zähnen und Kieferteilen zugewiesen und in ihrer Anzahl quantifiziert. Dieses Schema kann für die jeweiligen Behandlungssitzungen als Checkliste für den Zahnarzt und auch für das Praxispersonal dienen. Es erlaubt jedoch bereits davor eine technisch-inhaltlich qualifizierte und insbesondere zeitlich aufgelöste Übersicht über das gesamte Behandlungsprojekt im Sinne eines Arbeitsvorbereitungsplans. Im Übrigen kann das Computerprogramm natürlich auch so ausgestaltet sein, dass es mit Hilfe der vorliegenden Daten zu den entsprechenden Zeitpunkten bei der Rechnungserstellung hilft, beispielsweise am Ende des gesamten Behandlungsprojekts eine Gesamtrechnung für alle Sitzungen erstellt.

Das erfindungsgemäße Programm beschränkt sich also nicht auf eine buchhalterische Unterstützung sondern erlaubt eine hinsichtlich der Berücksichtigung zeitlicher Parameter technisch qualifizierte Arbeitsvorbereitung, insbesondere Terminplanung, Material- und Hilfsmittelbeschaffung, und die Erstellung von Checklisten und damit eine verbesserte Kontrolle der Arbeitsdurchführung und der Beschaffung.

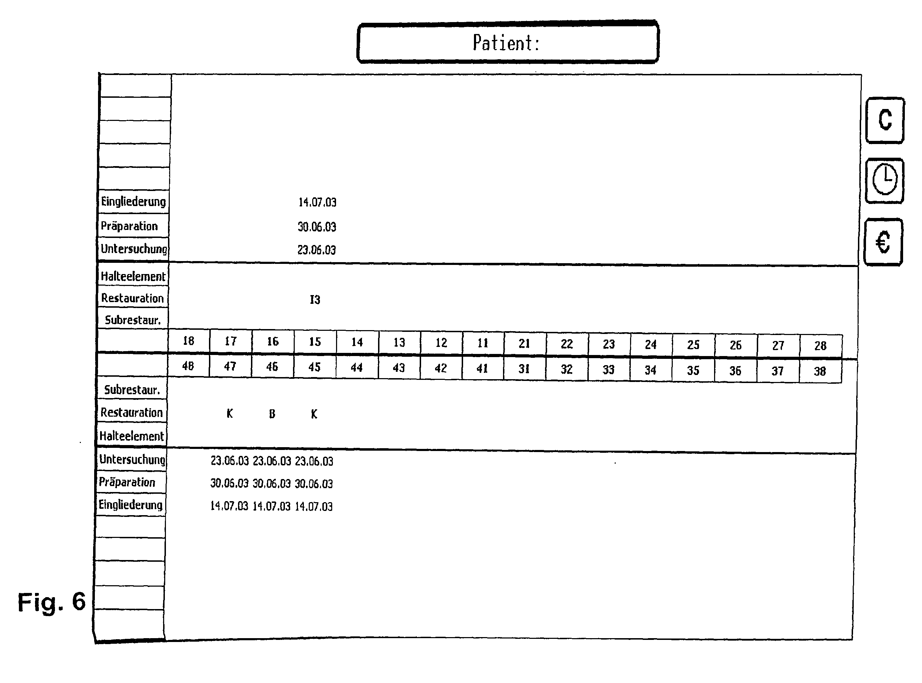

Fig. 6 zeigt zur Veranschaulichung einen Gesamtbildschirminhalt bei der Terminfestlegung entsprechend Fig. 5, wobei hier beide Kiefer des Patienten in Gänze dargestellt sind. In Fig. 6 sind von Fig. 5 abweichende Termine eingetragen und zusätzlich für den Zahn 15 im linken Oberkiefer für einen mit dem Planungskürzel 13 symbolisierten Teil des Behandlungsprojekts Maßnahmen für die drei Behandlungstermine am 23.06, 30.06. und 14.07.2003 festgelegt. Dieser Teil des Behandlungsprojekts soll nicht im Einzelnen dargestellt werden. Fig. 6 verdeutlicht, dass das Programm in sehr übersichtlicher Weise mit einer vertikalen Zeitachse und einer horizontalen Zahnzuordnung und den dem Zahnarzt und Praxispersonal vertrauten Planungskürzeln einen matrixartigen Überblick über das gesamte Behandlungsprojekt bietet.

Die im rechten Bereich mit C, dem Uhrensymbol und dem Eurosymbol beschriebenen Schaltflächen dienen zum Löschen von Eingaben, zur Terminfestlegung bzw. zum Umschalten in ein Buchhaltungsmodul zur Rechnungserstellung oder Erstellung eines Heil- und Kostenplans.

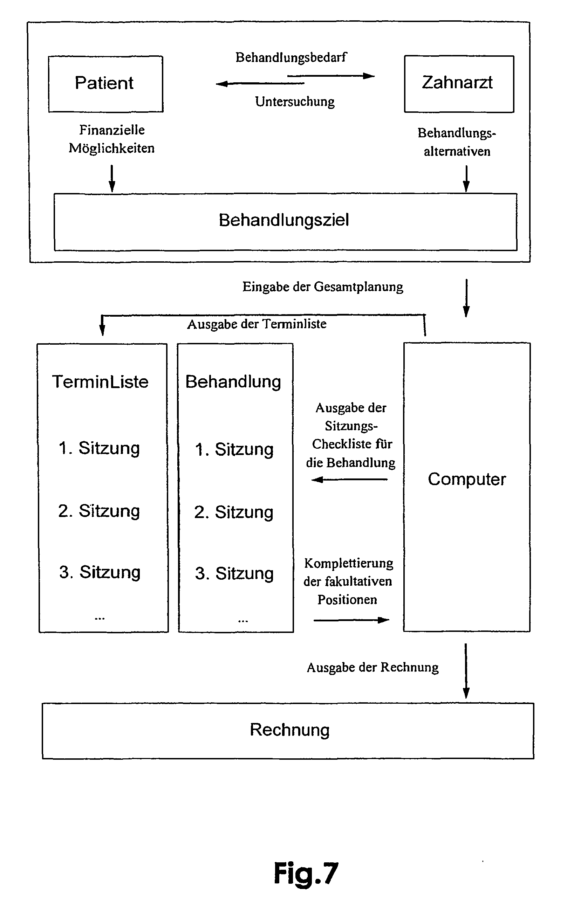

Fig. 7 dient zum Vergleich mit Fig. 1 und zeigt den Ablauf des Behandlungsprojekts in Übersichtsdarstellung. Der obere Kasten entspricht dabei Fig. 1 und muss daher nicht neu erläutert werden. Zu ergänzen bleibt allenfalls, dass dem Zahnarzt die Beratung des Patienten hinsichtlich seines Behandlungsbedarfs, der damit verbundenen Kosten und der zur Verfügung stehenden Alternativen durch die Übersichtsfunktion des Computerprogramms erleichtert wird. Es können also auch versuchsweise Behandlungsprojekte geplant werden, um die Beratung zu stützen und zu veranschaulichen. Nicht gewünschte Behandlungsprojekte können dann wieder gelöscht werden. In der beschriebenen Weise wird die eigentlich vorgesehene Behandlung als Behandlungsprojekt geplant, wie dies bereits anhand der Fig. 5 und 6 dargestellt wurde. Dabei können neben der Übersicht über die Gesamtplanung Terminlisten und Sitzungschecklisten für die einzelnen Behandlungssitzungen und auch andere Behandlungsteile, wie Laborarbeit, ausgegeben werden. Während des Ablaufs des Behandlungsprojekts können im Übrigen Änderungen und Rückmeldung über das Vorliegen oder Weglassen fakultativer Positionen erfolgen. Während des gesamten Behandlungsprojekts haben alle beteiligten Personen überdies die Möglichkeit sich über die Planung und Arbeitsvorbereitung zu informieren und ihre Arbeitsschritte dementsprechend einzurichten und zu überprüfen.

Am Ende des Behandlungsprojekts kann wiederum, wie im Stand der Technik auch, eine Gesamtabrechnung erfolgen. Allerdings kann hier die Rechnungserstellung zusätzlich eine zeitliche Differenzierung, also eine Zuordnung einzelner Rechnungsposten zu bestimmten Behandlungssitzungen vornehmen. Damit wird im Sinne einer Kostentransparenz und Glaubwürdigkeitssteigerung dem Patienten die Zuordnung der Kosten zu der erfolgten Behandlung besser nachvollziehbar.

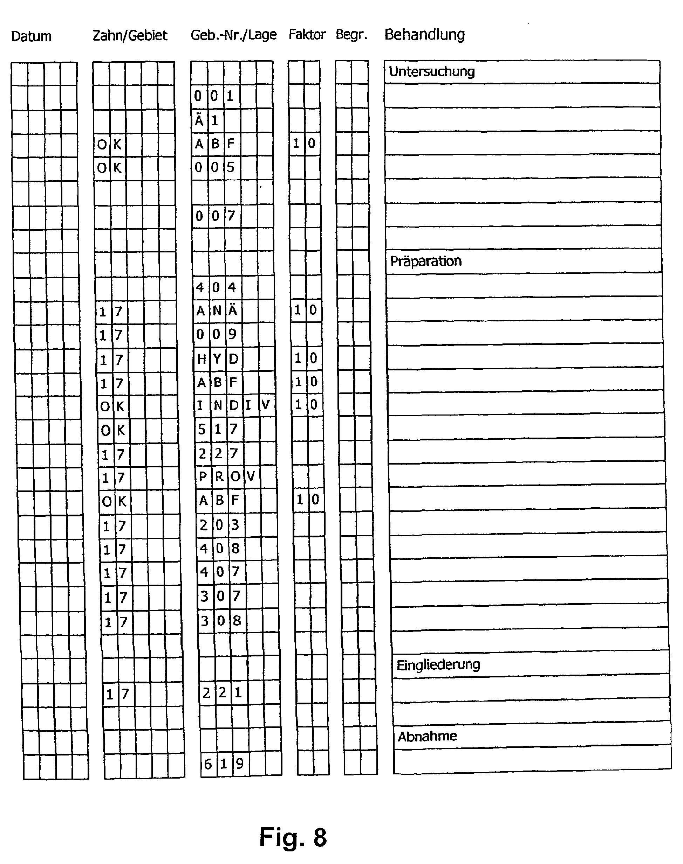

Die Figuren 8, 9 und 10 illustrieren den Erfindungsaspekt der Ausgabe eines Behandlungsablaufplanteils, hier zu vier Sitzungen mit dem Patienten, und der handschriftlichen Eingabe. Diese Illustrationen beziehen sich auf das anhand der Figuren 1-7 erläuterte Programm und Verfahren, legen jedoch einen etwas abweichenden zahnmedizinischen Fall zugrunde.

Figur 8 zeigt ein Karteiblatt mit einem oben rechts angegebenen Barcode. Dieses Karteiblatt kann entweder ein durch einen Computerdrucker ausgegebenes Karteiblatt im physikalischen Sinn oder der grafische Aufbau einer Monitoranzeige sein. Im letzteren Fall wäre der Barcode jedenfalls dann nicht notwendig, wenn nicht zusätzlich ein Ausdruck erfolgt. Im Folgenden wird der Einfachheit halber von einem Ausdruck ausgegangen, ohne dass dies Wesentliches an dem Ausführungsbeispiel ändert.

Der Ausdruck aus Figur 8 beinhaltet sechs vertikale Spalten, die jeweils zum besseren Verständnis eine Überschrift tragen. Die erste Spalte ist vorgesehen für die zeitlichen Daten der einzelnen Behandlungsschritte. Diese Spalte ist in der ausgegebenen Form leer. Sie zeigt, dass dennoch eine durch Zuordnungslinien gegebene Unterteilung in einzelne Felder vorgegeben ist.

Die zweite Spalte bezieht sich auf den von der Behandlung betroffenen Zahn oder das entsprechende Gebiet. Dabei bedeutet beispielsweise OK den ganzen Oberkiffer und 17 einen bestimmten Zahn (vgl. Figur 6). Diese Spalte ist bereits von dem Datenverarbeitungsgerät beschrieben, enthält also schriftliche Daten.

Die nächste dritte Spalte bezieht sich auf entsprechende, dem einzelnen Behandlungsschritt zugeordnete Gebührennummem aus verschiedenen Gebührenordnungen. Das Kürzel ABF bedeutet dabei beispielsweise Abformmaterial, das Kürzel HYD Hydrokolloid und das Kürzel INDIV einen individuellen Löffel sowie das Kürzel PROV ein Provisorium. Auch diese Spalte ist bereits bei der Ausgabe beschrieben. Der Begriff "Lage" in der dritten Spalte bedeutet, dass im Fall von Füllungen hier auch die Lage der Füllung erscheinen kann, die dann auch auf der Rechnung erscheint. In solchen Fällen kann aus der Lage bereits die Gebührennummer folgen, so dass die Angabe der Lage die Gebührennummer ersetzen kann. Es folgt eine Spalte mit entsprechenden Faktoren, die in diesem Fall ebenfalls durch das Programm vorgegeben sind. Dabei kann es sich um feste Vorgaben oder auch um in der erläuterten Weise anhand von geschätzten Zeit- und Materialaufwendungen ermittelte Faktoren handeln, die gegebenenfalls mit einer Patientenvereinbarung zu verknüpfen wären. Den eingetragenen Faktoren 1,0 sind Materialposten zugeordnet, bei denen sie ohnehin anzuwenden sind. Die übrigen Felder wären bei dieser Ausführungsform dann und nur dann vom Zahnarzt auszufüllen, wenn er von den Standardfaktoren 2,3 bei zahnärztlichen Leistungen bzw. 1,8 bei Röntgenleistungen abweichen will. Ohne Eintrag verwendet das Programm die entsprechenden Standardfaktoren. Bei diesem Ausführungsbeispiel liegt kein solcher abweichender Eintrag vor.

Die letzte Spalte ist vorgesehen für Behandlungsbemerkungen im Klartext. Sie ist ebenfalls bereits beschriftet, und zwar mit Stichworten zu den jeweiligen Sitzungen, die die Übersicht erleichtern. Im Übrigen hält das Programm bei dieser Ausführungsform diese letzte Spalte für Bemerkungen frei.

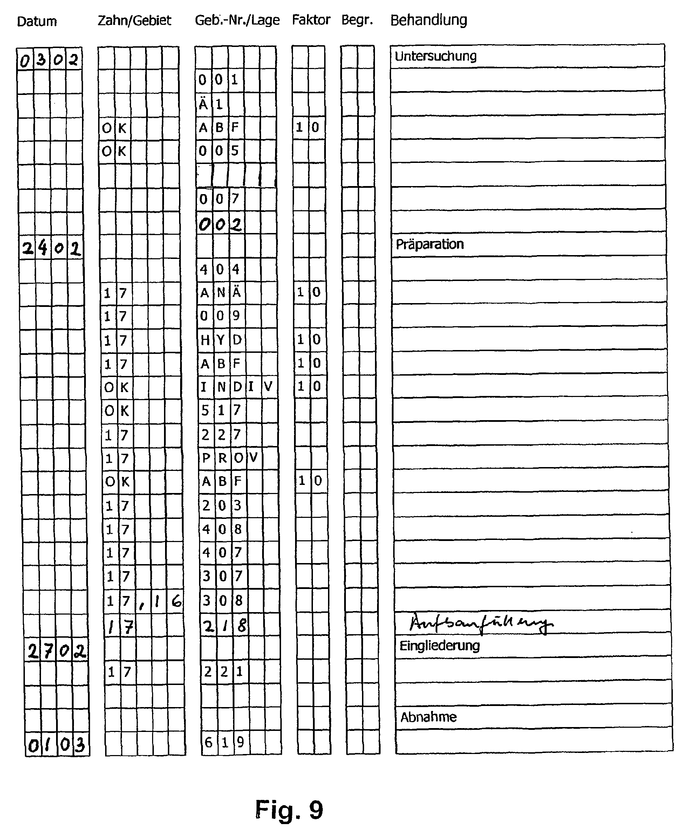

Der behandelnde Zahnarzt sieht diese Karteikarte vor und während der Behandlung vor sich und füllt sie in der in Figur 9 dargestellten Weise handschriftlich aus. Dies kann mit einem Kugelschreiber oder aber auch mit einem entsprechenden Stift auf einer sensorischen Unterlage, auf die die Karteikarte aufgelegt ist (und schließlich auch in Form einer Monitoranzeige als Karteikarte auf einem berührungsempfindlichen Monitor selbst (touch-screen)) erfolgen.

Man erkennt zunächst, dass in der nächsten Datumsspalte vor einem Block der Behandlungsschritte, also beispielsweise vor dem Block der Behandlungsschritte mit den Gebührennummern 001, Ä1, ABF, 005, 007, das Datum der entsprechenden Sitzung handschriftlich eingetragen ist, hier der 03.02. Dieser Eintrag bildet gleichzeitig eine Bestätigung der Vornahme der aufgezählten Behandlungsschritte. Weiterhin ist ein Behandlungsschritt 002 am Ende der Sitzung handschriftlich ergänzt.

In entsprechender Weise ist vor dem nächsten Block von Behandlungsschritten der 24.02., dann der 27.02. und schließlich der 01.03. eingetragen. Am Ende der Sitzung des 24.02. wurde der Behandlungsschritt mit der Gebührennummer 308 neben dem Zahn 17 auch für den Zahn 16 vorgenommen, was handschriftlich in der zweiten Spalte ergänzt ist. Ferner wurde auf dem Zahn 17 zusätzlich eine Aufbaufüllung mit der Gebührennummer 218 vorgenommen, die in der rechten Spalte mit den Behandlungskommentaren handschriftlich ergänzt ist. Bei diesem Beispiel handelt es sich um eine Einzelkrone, bei der dem ergänzten Eintrag entsprechend eine Aufbaufüllung notwendig wurde. Die medizinischen Einzelheiten sollen hier im Weiteren nicht vereinzelt werden, weil sie für das Prinzip der Erfindung nicht wesentlich sind.

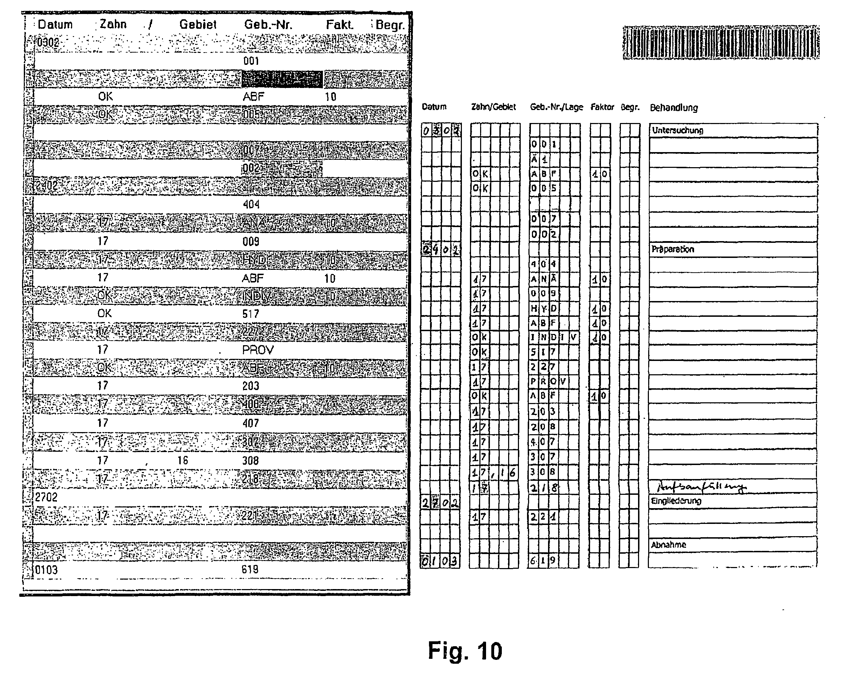

Figur 10 zeigt rechts die handschriftlich ergänzte Karteikarte aus Figur 9, die in dieser Form bereits eingescannt ist. Der Barcode oben rechts erlaubt eine direkte Dokumentenzuordnung. Im Übrigen erscheint auf dem Monitor die in Figur 10 links dargestellte Anzeige, die neben den ohnehin schon in Figur 8 vorgegebenen Inhalten auch die handschriftlich ergänzten Einzelheiten beinhaltet. Man erkennt, dass die rechte Spalte mit den Klartextkommentaren nicht miterfasst wurde, weil sie für das Programm nicht wesentlich ist.

Beispielsweise erkennt man in der Datumsspalte, dass die zweite und vierte Ziffer des ersten Datums, die erste Ziffer des zweiten Datums, die zweite Ziffer des vierten Datums und die erste Ziffer des vierten Datums sowie schließlich die zweite Ziffer 7 der handschriftlichen Ergänzung zur Aufbaufüllung mit einer gewissen Unsicherheit behaftet waren und das Programm eine Überprüfung anregt, indem es diese Ziffern unterlegt. Der Vergleich mit dem linken Teil der Figur 10 zeigt aber, dass die Erkennung zutreffend war. Das Programm wertet im Übrigen die Daten als zahnärztliche Bestätigung der entsprechenden Sitzung, also der nachfolgenden Behandlungsschritte.

Die Figuren 8-10 zeigen also eine EDV-lesbare Karteikarte im Bereich der zahnärztlichen Behandlung, die einen auf mehrere Sitzung bezogenen Teil des Behandlungsplans oder auch den gesamten Behandlungsplan enthält. Die von der EDV nicht erfasste Behandlungsbemerkungsspalte dient nur zur Orientierung des Zahnarztes und zur ausführlicheren Dokumentation in Papierform. Während der Behandlung wird die Karteikarte im Übrigen in den weiteren Spalten mit den aktuellen Sitzungsdaten und zusätzlich anfallenden Leistungen ergänzt und gegebenenfalls korrigiert. Mit dieser Vorgehensweise kann faktisch eine Lege Artis-Behandlung in einer Iso-9000-Umgebung vorgezeichnet werden. Beispielsweise werden dabei notwendige Vitalitätsproben oder Röntgenkontrollen vor einer Präparation nicht mehr vergessen.

## Patentansprüche

1. Verfahren zur zahnärztlichen Arbeitsvorbereitung mit dem Schritt:
- Aufnahme von Daten zu einem zahnärztlichen Behandlungsprojekt in ein Datenverarbeitungsgerät,
**gekennzeichnet durch** die zusätzlichen Schritte:
- Abrufen von vorbestimmten Ablauftabellendaten aus einem Ablauftabellenspeicher des Datenverarbeitungsgeräts, welche Ablauftabellendaten den Behandlungsprojektdaten zugeordnet sind und technische Details im Sinne vorbestimmter zeitlicher Parameter zu zu dem Behandlungsprojekt gehörenden Behandlungsschritten enthalten, welche vorbestimmten zeitlichen Parameter die zeitliche Reihenfolge sowie Mindest- und/oder Maximalzeitabstände von Behandlungsschritten in dem Behandlungsprojekt wiedergeben,
Erstellen eines Behandlungsablaufplans aus den Ablauftabellendaten, welcher eine Mehrzahl Behandlungseinheiten mit jeweiligen Terminen enthält, wobei den Behandlungseinheiten jeweilige Behandlungsschritte zugeordnet sind und die Zuordnung der Behandlungsschritte zu den Behandlungseinheiten und den Terminen die in den Ablauftabellendaten enthaltenen vorbestimmten zeitlichen Parameter berücksichtigt.

2. Verfahren nach Anspruch 1, bei dem die zeitlichen Parameter Mindestzeitabstände zwischen Behandlungsschritten wiedergeben.

3. Verfahren nach Anspruch 1 oder 2, bei dem die zeitlichen Parameter Maximalzeitabstände zwischen Behandlungsschritten wiedergeben.

4. Verfahren nach einem der vorstehenden Ansprüche, bei dem in den Behandlungsprojektdaten Daten zu nichtzahnärztlichen Tätigkeiten, etwa Labor, Herstellung von Vorrichtungen oder Beschaffung von Materialien und/oder Hilfsmitteln, enthalten sind.

5. Verfahren nach einem der vorstehenden Ansprüche, bei dem der Behandlungsablaufplan durch einen Benutzer interaktiv variiert wird.

6. Verfahren nach Anspruch 5, bei dem die Variationen des Behandlungsablauf plans hinsichtlich der Einhaltung der in den Ablauftabellendaten enthaltenen zeitlichen Parameter kontrolliert werden und im Fall von Nichteinhaltung zumindest eine Sperrung der Variation oder eine Wamanzeige des Datenverarbeitungsgeräts erfolgt.

7. Verfahren nach Anspruch 6, bei dem der Behandlungsablaufplan im Laufe der Ausführung des Behandlungsprojekts ansprechend auf dabei auftretende Untersuchungs-/Behandlungsergebnisse variiert wird.

8. Verfahren nach einem der vorstehenden Ansprüche, bei dem Behandlungssitzungschecklisten erstellt und ausgegeben werden

9. Verfahren nach einem der vorstehenden Ansprüche, bei dem Terminmerkblätter erstellt und ausgegeben werden.

10. Verfahren nach einem der vorstehenden Ansprüche, bei dem die Behandlungs, projektdaten zahnärztlichen Gebührenordnungsziffem zugeordnet werden.

11. Verfahren nach Anspruch 10, bei dem das Datenverarbeitungsgerät selbsttätig Rechnungsvorschläge einschl. Laborrechnungsvorschläge erstellt.

12. Verfahren nach Anspruch 11, bei dem das Datenverarbeitungsgerät selbsttätig Kostenpläne erstellt.

13. Verfahren nach einem der vorstehenden Ansprüche mit den zusätzlichen Schritten:
- Ausgabe zumindest eines Teils des Behandlungsablaufplans mit einzelnen Behandlungsschritten,
- Eingabe von Daten zu dem ausgegebenen Teil des Behandlungsablaufplans durch handgeführte Schrift auf dem ausgegebenen Teil des Behandlungsablaufplans.

14. Verfahren nach Anspruch 13, bei dem die Ausgabe als Ausdruck außerhalb eines zahnärztlichen Behandlungsraum erfolgt.

15. Verfahren nach Anspruch 13 oder 14, bei dem die Ausgabe als Monitoranzeige erfolgt und die Eingabe von Daten auf dem ausgegebenen Teil des Behandlungsablaufplans durch handgeführte Schrift auf der Monitoranzeige erfolgt.

16. Verfahren nach Anspruch 13 oder 14, bei dem die Ausgabe als Ausdruck erfolgt und die Eingabe von Daten auf dem ausgegebenen Teil des Behandlungsablaufplans durch Handschrift auf dem Ausdruck und Einscannen des handbeschriebenen Ausdrucks sowie elektronische Erkennung der Handschrift erfolgt.

17. Verfahren nach Anspruch 15, bei dem der Ausdruck einen Barcode enthält und der Barcode den ausgedruckten Teil oder den Behandlungsablaufplan insgesamt identifiziert.

18. Verfahren nach Anspruch 16 oder 17, bei dem der Ausdruck einen durch das Einscannen und die Handschrifterkennung nicht erfassten Bereich enthält, der handbeschriftet wird.

19. Verfahren nach einem der Ansprüche 16-18, bei dem der Ausdruck einen tabellarischen Aufbau mit Zuordnungslinien aufweist und die Zuordnungslinien bei dem Einscannen zur Mittenbestimmung und/oder Ausrichtung des Ausdrucks verwendet werden.

20. Verfahren nach einem der Ansprüche 13-19, bei dem nach der Dateneingabe durch handgeführte Schrift eine Monitoranzeige Schrifterkennungsunsicherheiten anzeigt.

21. Verfahren nach einem der Ansprüche 13-20, bei dem Behandlungsschritte des ausgegebenen Teils des Behandlungsablaufplans von einem Zahnarzt im Rahmen der Dateneingabe durch handgeführte Schrift bestätigt werden.

22. Verfahren nach Anspruch 21, bei dem die Ausgabe des Teils des Behandlungsablaufplans bei zu bestätigenden Behandlungsschritten deren Datum offen lässt und die Bestätigung durch den Zahnarzt eine handschriftliche Datumseingabe beinhaltet.

23. Verfahren nach einem der vorstehenden Ansprüche, bei dem durch das Datenverarbeitungsgerät mit Hilfe der Ablauftabellendaten für das Behandlungsprojekt Zeit- und Materialaufwandsschätzungen durchgeführt werden und darauf aufbauend Abrechnungsfaktoren für Gebührenordnungsziffem ermittelt werden.

24. Verfahren nach Anspruch 23, bei dem nach Ermittlung der Abrechnungsfaktoren bei Überschreiten bestimmter Grenzwerte automatisch Vordrucke für Patientenvereinbarungen ausgedruckt werden.

25. Computerprogrammprodukt, das so ausgelegt ist, dass nach Laden des Computerprogramms in ein Datenverarbeitungsgerät dieses an die Durchführung des Verfahrens nach einem der vorstehenden Ansprüche angepasst ist.

26. Datenverarbeitungsgerät, in dem ein Computerprogramm gemäß Anspruch 25 geladen ist und das demzufolge an die Durchführung des Verfahrens nach einem der Ansprüche 1 - 24 angepasst ist.

## Claims

1. A method for dental work scheduling including the step:
- recording of data of a dental treatment project in a data processing apparatus,
**characterized by** the additional steps:
- fetching predetermined operation schedule table data from an operation schedule table memory of said data processing apparatus, which operation schedule table data are allocated to said treatment project data and comprise technical details in terms of predetermined time parameters of treatment steps belonging to said treatment project, said predetermined time parameters representing a time sequence as well as minimum and/or maximum time interspaces of treatment steps in said treatment project,
- producing a treatment operation schedule plan of said operation schedule table data, said plan comprising a plurality of treatment units having respective time dates, wherein respective treatment steps are allocated to said treatment units and said allocation of said treatment steps to said treatment units and to said time dates is adapted to said predetermined time parameters comprised in said operation schedule table data.

2. The method of claim 1 wherein said time parameters are minimum time interspaces between treatment steps.

3. The method of claim 1 or 2 wherein said time parameters are maximum time interspaces between said treatment steps.

4. The method of one of the preceding claims in which data of non-dental operations are included in said treatment project data, such as laboratory, production of devices, or provision of material and/or auxiliary means.

5. The method of one of the preceding claims in which said treatment operation schedule plan is interactively varied by a user.

6. The method of claim 5 wherein the variations of the treatment operation schedule plan are monitored with regard to said time parameters comprised in said operation schedule table data, and in case of non-compliance at least a blocking of said variation or an alert signal of said data processing apparatus results.

7. The method of claim 6 in which said treatment operation schedule plan is varied during the execution of said treatment project in response to examination or treatment results occurring therein.

8. The method of one of the preceding claims in which treatment session check lists are produced and output.

9. The method of one of the preceding claims in which time date reminder sheets are produced and output.

10. The method of one of the preceding claims in which said treatment project data are allocated to dentist fee schedule items.

11. The method of claim 10 in which said data processing apparatus autonomously produces debit note proposals including laboratory debit note proposals.

12. The method of claim 11 in which said data processing apparatus autonomously produces cost plans.

13. A method of one of the preceding claims including the additional steps:
- outputting of at least a part of said treatment operation schedule plan including individual treatment steps,
- inputting of data relating to said output part of said treatment operation schedule plan by a hand-guided writing on said output part of said treatment operation schedule plan.

14. The method of claim 13 wherein said output is done by printing outside of a dental treatment room.

15. The method of claim 13 or 14 in which said outputting is done by a monitor display and said inputting of data is done on said output part of said treatment operation schedule plan by hand-guided writing on said monitor display.

16. The method of claim 13 or 14 in which said outputting is done by printing and said inputting of data is done on said output part of said treatment operation schedule plan by handwriting on the print and scanning the hand-written print as well as electronic recognition of said handwriting.

17. The method of claim 16 in which said print comprises a bar code and said bar code identifiers said printed part or the whole of said treatment operation schedule plan.

18. The method of claim 16 or 17 in which said print comprises a region not included in said scanning and in said handwriting recognition, said region being written by hand.

19. The method of one of claims 16 - 18 in which said print comprises a tabulated arrangement including allocation lines and said allocation lines are used for a centre definition and/or an orientation of said print during scanning.

20. The method of one of claims 13 - 19 in which a monitor display displays handwriting-recognition uncertainties after said data input by said hand-guided writing.

21. The method of one of claims 13 - 20 in which said treatment steps of said output part of said treatment operation schedule plan are confirmed by a dentist during said data input by said hand-guided writing.

22. The method of claim 21 in which said outputting of said part of said treatment operation schedule plan leaves open a time date thereof in case of treatment steps to be confirmed and wherein said confirmation by said dentist comprises a handwritten time date input.

23. The method of one of the preceding claims wherein said data processing apparatus conducts estimations of expenditures of time and material by means of said operation schedule table data for said treatment project and wherein billing factors for fee schedule items are determined on this basis.

24. The method of claim 23 in which blank prints for patient agreements are printed automaticaily after the determination of said billing factors in case of exceeding certain limits.

25. A computer program product adapted such that after loading said computer program into a data processing apparatus, said data processing apparatus is adapted to executing the method of one of the preceding claims.

26. A data processing apparatus in which a computer program according to claim 25 is loaded and which is thus adapted to executing the method of one of claims 1 -24.

## Revendications

1. Procédé pour la préparation du travail dentaire avec l'étape :
- d'enregistrement de données d'un projet de traitement dentaire dans un appareil de traitement de données,
**caractérisé par** les étapes supplémentaires :
- extraction de données prédéfinies d'un tableau de déroulement d'une mémoire de tableaux de déroulement de l'appareil de traitement de données, lesquelles données de tableau de déroulement sont associées aux données d'un projet de traitement et contiennent des détails techniques dans le sens de paramètres temps prédéfinis des étapes de traitement qui font partie du projet de traitement, lesquels paramètres temps prédéfinis restituent la séquence dans le temps ainsi que des intervalles de temps minimaux et/ou maximaux des étapes de traitement dans le projet de traitement,
- établissement d'un plan de déroulement du traitement à partir des données du tableau de déroulement qui contient une multitude d'unités de traitement avec des délais respectifs, des étapes de traitement étant associées à chacune des unités de traitement et l'association des étapes de traitement aux unités de traitement et aux délais tenant compte des paramètres temps prédéfinis contenus dans les données des tableaux de déroulement.

2. Procédé selon la revendication 1 dans lequel les paramètres temps restituent des intervalles de temps minimaux entre des étapes de traitement.

3. Procédé selon la revendication 1 ou 2 dans lequel les paramètres temps restituent des intervalles de temps maximaux entre des étapes de traitement.

4. Procédé selon l'une des revendications précédentes dans lequel des données d'activité non dentaire, comme laboratoire, fabrication de dispositif ou approvisionnement en matériaux et/ou en moyens auxiliaires, sont contenues dans les données du projet de traitement.

5. Procédé selon l'une des revendications précédentes dans lequel un utilisateur fait varier le plan de déroulement du traitement de manière interactive.

6. Procédé selon la revendication 5 dans lequel les variations du plan de déroulement du traitement sont contrôlées pour ce qui est du respect des paramètres temps contenus dans les données du tableau du déroulement et qu'en cas de non-respect il se produit au moins un blocage de la variation ou un affichage d'avertissement de l'appareil de traitement des données.

7. Procédé selon la revendication 6 dans lequel le plan de déroulement du traitement est varié au cours de la réalisation du projet de traitement en réponse aux résultats d'examens/de traitement qui interviennent au cours de celui-ci.

8. Procédé selon l'une des revendications précédentes dans lequel des listes de contrôle des séances du traitement sont établies et éditées.

9. Procédé selon l'une des revendications précédentes dans lequel des aides- mémoires de délais sont établis et édités.

10. Procédé selon l'une des revendications précédentes dans lequel les données du projet de traitement sont associées à des indices de barèmes d'honoraires dentaires.

11. Procédé selon la revendication 10 dans lequel l'appareil de traitement de données établit automatiquement des propositions de factures ainsi que des propositions de factures de laboratoires.

12. Procédé selon la revendication 11 dans lequel l'appareil de traitement des données établit automatiquement des plans de coûts.

13. Procédé selon l'une des revendications précédentes avec les étapes supplémentaires :
- édition d'au moins une partie du plan de déroulement du traitement avec des étapes individuelles de traitement.
- entrée de données sur la partie éditée du plan de déroulement du traitement par écriture à la main sur la partie éditée du plan de déroulement du traitement.

14. Procédé selon la revendication 13 dans lequel l'édition est effectuée en tant que sortie par typo en dehors d'un espace de traitement dentaire.

15. Procédé selon la revendication 13 ou 14 dans lequel l'édition est effectuée en tant qu'affichage sur l'écran et l'entrée de données sur la partie éditée du plan de déroulement du traitement est effectuée par écriture à la main sur l'affichage sur l'écran.

16. Procédé selon la revendication 13 ou 14 dans lequel l'édition est effectuée en tant que sortie par typo et l'entrée de données sur la partie éditée du plan de déroulement du traitement est effectuée par écriture à la main sur la sortie typo et par balayage par scanner de la sortie typo sur laquelle il a été écrit à la main ainsi que par reconnaissance électronique de l'écriture à la main.

17. Procédé selon la revendication 16 dans lequel la sortie par typo contient un code barres et le code barres identifie la partie imprimée ou le plan de déroulement du traitement dans son ensemble.

18. Procédé selon la revendication 16 ou 17 dans lequel la sortie typo contient une zone non saisie par le balayage du scanner et la reconnaissance de l'écriture à la main, zone qui est écrite à la main.

19. Procédé selon l'une des revendications 16 à 18 dans lequel la sortie typo présente une structure de tableau avec des lignes de correspondance et les lignes de correspondance sont utilisées lors du balayage par scanner pour la détermination du milieu et/ou l'alignement de la sortie typo.

20. Procédé selon l'une des revendications 13 à 19 dans lequel, après l'entrée des données par écriture à la main, un affichage sur l'écran affiche des incertitudes de reconnaissance d'écriture.

21. Procédé selon l'une des revendications 13 à 20 dans lequel des étapes de traitement de la partie éditée du plan de déroulement du traitement sont confirmées par un dentiste par écriture à la main dans le cadre de l'entrée des données.

22. Procédé selon la revendication 21 dans lequel, pour des étapes de traitement à confirmer, l'édition de la partie du plan de déroulement du traitement laisse libre leur date et la confirmation par le dentiste contient une entrée de date écrite à la main.

23. Procédé selon l'une des revendications précédentes dans lequel des évaluations de temps à consacrer et de dépenses de matériaux sont exécutées par l'appareil de traitement de données à l'aide de données du tableau de déroulement pour le projet de traitement et des facteurs de facturation sont déterminés sur cette base pour des indices du barème d'honoraires.

24. Procédé selon la revendication 23 dans lequel des formulaires pour des conventions avec les patients sont automatiquement imprimés après détermination des facteurs de facturation lors du dépassement de certains seuils.

25. Produit de programme d'ordinateur qui est conçu de telle manière qu'après le chargement du programme d'ordinateur dans un appareil de traitement de données celui-ci est adapté à la réalisation du procédé selon l'une des revendications précédentes.

26. Appareil de traitement des données dans lequel un programme d'ordinateur est selon la revendication 25 est chargé et qui est par conséquent adapté à la réalisation du procédé selon l'une des revendications 1 à 24.
